(19) Europäisches Patentamt
European Patent Office
Office européen des brevets

(11) EP 0 710 226 B1

# (12) FASCICULE DE BREVET EUROPEEN

(45) Date de publication et mention de la délivrance du brevet:
**14.10.1998 Bulletin 1998/42**

(21) Numéro de dépôt: **94922288.9**

(22) Date de dépôt: **21.07.1994**

(51) Int Cl.6: **C07C 323/59**, C07C 323/60, C08G 69/42, C08G 75/14, A61L 17/00, A61L 25/00, A61K 9/22

(86) Numéro de dépôt international:
**PCT/FR94/00914**

(87) Numéro de publication internationale:
**WO 95/03272 (02.02.1995 Gazette 1995/06)**

## (54) NOUVEAUX PRODUITS ORGANIQUES CONTENANT DES FONCTIONS THIOLS REACTIVES, L'UN DE LEURS PROCEDES DE PREPARATION ET LES BIOMATERIAUX LES CONTENANT

NEUE REAKTIVE THIOL-GRUPPE ENTHALTENDE ORGANISCHE PRODUKTE, EIN VERFAHREN ZU IHRER HERSTELLUNG UND SIE ENTHALTENDE BIOMATERIALIEN

NOVEL ORGANIC PRODUCTS CONTAINING REACTIVE THIOL FUNCTIONS, ONE METHOD FOR PREPARING SAME, AND BIOMATERIALS CONTAINING SAID PRODUCTS

(84) Etats contractants désignés:
**AT BE CH DE DK ES FR GB GR IE IT LI LU MC NL PT SE**

(30) Priorité: **21.07.1993 FR 9309198**

(43) Date de publication de la demande:
**08.05.1996 Bulletin 1996/19**

(73) Titulaire: **FLAMEL TECHNOLOGIES**
**F-69693 Venissieux Cédex (FR)**

(72) Inventeurs:
- **CONSTANCIS, Alain**
  **F-69008 Lyon (FR)**
- **SOULA, Gérard**
  **F-69330 Meyzieu (FR)**

(74) Mandataire: **Fleurance, Raphael**
**Cabinet Beau de Loménie,**
**51, Avenue Jean Jaurès,**
**B.P. 7073**
**69301 Lyon Cédex 07 (FR)**

(56) Documents cités:
**EP-A- 0 332 530**
**FR-A- 1 441 235**
**FR-A- 2 159 183**
**FR-A- 2 274 605**

- **CHEMICAL ABSTRACTS, vol. 102, no. 8, 25 Février 1985, Columbus, Ohio, US; abstract no. 62648a, R.D. KATSARAVA, et al.: 'Heterochain polymers from natural amino acids. Oxazolinone method for the synthesis of polyamides containing bonds cleavable by fermentation in the main chain' page 6 ; & ACTA POLYMERICA, 1985, 36(1), 29-38**

EP 0 710 226 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention conceme de nouveaux produits organiques résultant, par exemple, de la condensation d'un diacide carboxylique avec un acide aminé soufré ou l'un de ses dérivés. Ces produits comportent des fonctions thiols SH réactives pouvant s'oxyder pour former des ponts disulfures, conduisant à des polymères, réticulés ou non.

## TECHNIQUE ANTERIEURE :

L'une des applications visée par l'invention est l'emploi de ces nouveaux produits organiques et/ou de ces polymères réticulés ou non à titre de biomatériaux. Ces produits pourraient, par exemple, servir de matières premières pour la réalisation de systèmes de libération contrôlée de principes actifs, d'adhésifs biologiques, de sutures, de ligatures, de prothèses chirurgicales ou autres structures exogènes susceptibles de se substituer, au moins en partie, aux fonctions, notamment physiques ou mécaniques, des organes et des tissus.

On connaît déjà des polymères et des oligomères synthétiques biodégradables, qui sont constitués, très souvent, d'enchaînements simples et hydrolysables (esters ou amides) de composés propres à former des métabolites.

Ainsi, la demande de brevet **EP 0 332 530** décrit des polymères hydrophiles, de degré de polymérisation inférieur à 1 000, de préférence compris entre 20 et 300, et constitués par des polyamides résultant de la condensation de l'acide citrique avec des diamines, tels que la lysine, la cystamine, la cystine.

La synthèse de ces polyamides présente des difficultés réelles liées à la protection puis à la déprotection de l'acide citrique.

Ces polyamides biodégradables sont utilisables pour la préparation de vecteurs de médicaments, de sutures, de ligatures ou de prothèses, ou bien encore d'adhésifs chirurgicaux.

Si pour certaines applications, l'utilisation de polymères de masses relativement élevées, du type de ceux décrits dans la demande de brevet **EP 0 332 530**, est intéressante, pour d'autres usages, l'emploi de monomères ou d'oligomères porteurs de fonctions réactives ou polymérisables (prépolymères) est préférable. C'est le cas, en particulier, en chirurgie réparatrice (comblement osseux, ciments chirurgicaux, adhésifs biologiques ...) ou en chirurugie dentaire (ciments dentaires...). Dans ces applications, il est intéressant que le monomère ou prépolymère puisse diffuser très facilement dans le tissu à réparer et pénétrer ainsi dans toutes les cavités. La polymérisation peut ensuite intervenir "in situ" et donner naissance à un enchevêtrement de chaînes polymères ayant les propriétés de comblement, de cohésion ou d'adhésion désirées.

Dans cet état de la technique, l'un des objectifs essentiels de l'invention est de fournir des produits synthétiques organiques, qui puissent, notamment, servir de base à la préparation de biomatériaux atoxiques biocompatibles, biofonctionnels et utiles, entre autres, comme systèmes de libération contrôlée de principes actifs, sutures, ligatures, adhésifs chirurgicaux ou bien encore comme prothèses chirurgicales ou implants.

Un autre objectif essentiel de l'invention est de foumir des produits synthétiques organiques qui se trouvent sous forme de prépolymères et/ou de monomères, aptes à diffuser aisément dans les tissus biologiques et à polymériser in situ, voire in vivo, pour assurer, de façon satisfaisante, les fonctions de comblement, de renfort de la cohésion ou d'adhésion.

## EXPOSE DE L'INVENTION :

Ces objectifs et d'autres sont atteints par la présente invention qui concerne, en premier lieu, un nouveau produit organique contenant au moins deux fonctions thiols ou dérivés et/ou des fonctions carboxyliques, protégées ou non, et/ou des fonctions carbonylées, de formule générale suivante :

(I)

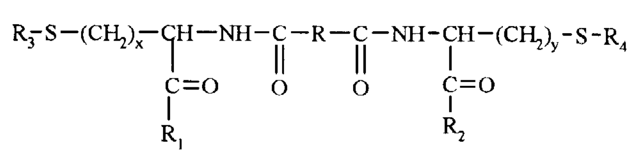

dans laquelle :

- R est une chaîne hydrocarbonée, de préférence alkylée comportant de 1 à 50 atomes de carbone et, plus préférentiellement encore, aliphatique, ayant de 1 à 10 atomes de carbone,
- $R_1$ et $R_2$ sont identiques ou différents et sont choisis parmi les groupements suivants :
  $-O-R_5$ ;

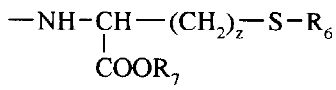 ;

$$—NH—(CH_2)_y—S-R_6$$

- $R_3$, $R_4$, $R_5$, $R_6$ et $R_7$ représentent, indépendamment, l'hydrogène ou un groupe aliphatique et/ou alicyclique et/ou aromatique, de préférence un groupe alkyle comprenant de 1 à 6 atomes de carbone et/ou un groupe aromatique et, plus préférentiellement encore, l'un des groupements suivants :

  $-CH_3$ ; $-CH_2CH_3$ ;

  $$—CH_2—Phe$$ ;

  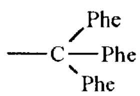

- x, y, z = 1 ou 2,

**à l'exclusion** des produits de formule (I) dans lesquels :

- R = $-(CH_2)_t$ avec t = 2,4 ou 6 à 12, ou phénylène,
- $R_1$ et $R_2$ sont identiques et correspondent à OH, - O - alkyle, ou - N - alkyle,
- $R_3$ et $R_4$ sont identiques et représentent l'hydrogène, - $CH_3$ ou $-CH_2$ - COOH,
- x et y sont identiques et sont égaux à 1 ou 2.

Pour des raisons de simplification, on désigne les cycles aromatiques par Phe dans tout le présent exposé.

Au sens de la présente invention, le terme "alkyle inférieur" désigne des radicaux comprenant de 1 à 6 atomes de carbone.

## MEILLEURE MANIERE DE REALISER L'INVENTION :

Les composés biologiques répondant à cette formule ont, avantageusement, une masse moléculaire relativement faible (inférieure à 2 000) et peuvent donc diffuser rapidement et aisément à travers les réseaux de protéines (collagène, élastine ...) ou glycoprotéines constituant les tissus. C'est une propriété qu'il est intéressant d'exploiter dans le domaine des adhésifs.

Une première sous-famille des produits de l'invention comprend ceux dans lesquels les radicaux $R_1$ et $R_2$ représentent $OR_5$.

Plus précisément encore, dès lors que $R_3$ et $R_4$ correspondent à l'hydrogène, on est en présence d'un oligomère présentant, à chacune de ses deux extrémités, une fonction SH portée par un motif cystéine ou dérivé (oligomère "di **SH**").

Ces fonctions SH ont la faculté de pouvoir réagir entre elles, pour former des ponts disulfures et permettre l'obtention de longues chaînes. Cette propriété peut être exploitée pour préparer des fils, films ou des solutions visqueuses biodégradables.

La présence de fonctions carboxyliques sur ces composés di **SH**, permet d'envisager des interactions avec d'autres molécules (par exemple macromolécules naturelles). Ceci va dans le sens de l'amélioration des propriétés adhésives. En outre, ces fonctions carboxyliques amènent un caractère hydrophile et une capacité à fixer des principes actifs.

Une deuxième sous-famille typique des produits conformes à l'invention regroupe les produits répondant à la formule générale sus-indiquée, dans laquelle le radical $R_1$ représente :

$$\begin{array}{l} -NH-CH-(CH_2)_z-S-R_6 \\ \quad\quad\;\; | \\ \quad\quad COOR_7 \end{array}$$

et $R_2$ représente - O - $R_5$ ou inversement.

Dès lors que $R_5$ et $R_6$ sont constitués par l'hydrogène, on peut qualifier ces composés d'oligomères "tri SH". Ces oligomères, dont les extrémités SH sont susceptibles de réagir pour former des ponts disulfure, laissent entrevoir des possibilités d'élaboration de réseaux multidirectionnels, qui ne peuvent qu'améliorer les propriétés mécaniques, les vertus d'adhésion et la résistance à la biodégradation des produits selon l'invention.

Une troisième sous-famille de produits organiques selon l'invention est constituée par les produits dans lesquels les radicaux $R_1$ et $R_2$ sont constitués par le radical :

$$\begin{array}{l} -NH-CH-(CH_2)_z-S-R_6 \\ \quad\quad\;\; | \\ \quad\quad COOR_7 \end{array}$$

Avec $R_3$, $R_4$ et $R_6$ correspondant à l'hydrogène, on définit un oligomère tétra fonctionnel, comportant quatre motifs SH à ses extrémités (oligomère "tétra SH"). Cette multiplicité de points d'ancrage potentiels est avantageusement exploitable dans le domaine des biomatériaux. Cela s'inscrit dans le prolongement de ce qui a été indiqué ci-dessus pour les oligomères di et tri-fonctionnels.

Le motif cystéique mis en oeuvre peut être formé par la cystéine en elle-même : x, y, z = 1 ou par l'homocystéine : x, y, z = 2, qui peuvent être, éventuellement, issus de cystine ou d'homocystine.

La chaîne alkylée R, éventuellement substituée, définit le radical :

$$\begin{array}{ccccc} -C & - & R & - & C- \\ \| & & & & \| \\ O & & & & O \end{array}$$

dans la formule (I), de telle sorte qu'il appartienne à la famille des restes d'acides poly, avantageusement di, carboxyliques, à l'exclusion de l'acide citrique, R étant, de préférence, sélectionné parmi les groupements suivants :

$-(CH_2)_p-$ ;

$$\begin{array}{l} -CH-(CH_2)_q \\ \;\;\; | \\ \;\; NH_2 \end{array} \quad ; \quad \begin{array}{l} -CH-(CH_2)_r- \\ \;\;\; | \\ \;\; OH \end{array}$$

avec :

- $p \le 5$, de préférence, égal à 2 (acide succinique) ou à 3 (acide glutarique),
- $q \le 5$, de préférence, égal à 1 (acide aspartique) ou à 2 (acide glutamique),
- et enfin, $r \le 5$, de préférence, égal à 1 (acide malique).

R peut être, également, composé d'enchaînements polylactique et/ou polyglycolique et/ou polyaminoacide, de faible masse moléculaire.

Ces oligomères conformes à l'invention sont porteurs de fonctions SH leur conférant des aptitudes à la polymérisation et/ou à la réticulation, éventuellement en présence d'un agent oxydant. Ils permettent donc d'obtenir, après

oxydation, des polymères, réticulés ou non, utilisables comme biomatériaux et éventuellement dégradables en métabolites naturels, i. e. qui interviennent dans les cycles biologiques des mammifères.

Par ailleurs, leur taille et leur structure sont telles qu'ils peuvent facilement migrer et pénétrer dans les tissus biologiques.

Il s'ensuit que ces oligomères peuvent accéder sans difficulté jusqu'aux sites biologiques visés et polymériser "in situ" de manière à former un enchevêtrement et/ou un réseau de chaines polymères.

Ces oligomères trouvent donc des débouchés en tant que constituants de produits de chirurgie réparatrice (comblement osseux, ciments chirurgicaux, adhésifs biologiques ...) ou de chirurgie dentaire (ciments dentaires ...) où l'on exploite leurs propriétés de comblement, de renfort de la cohésion ou d'adhésion.

Ces propriétés peuvent, également, présenter un intérêt dans un autre domaine d'application qui est celui des formes bioadhésives utilisées dans certains systèmes de libération contrôlée de principes actifs thérapeutiques. Pour certaines applications, il est préférable d'employer une forme liquide, car cela facilite l'application de l'ensemble principe actif-prépolymère, puis la formation d'une matrice polymère in situ au contact de la muqueuse (en présence ou non d'un agent oxydant déclenchant la polymérisation) permet une libération contrôlée du principe actif. De tels systèmes sont particulièrement avantageux pour le traitement d'affections locales.

De plus, le polymérisation des produits selon l'invention par oxydation des **SH** en ponts disulfures peut, également, être effectuée in vitro et permettre ainsi la formation de films ou d'objets moulables utilisables en tant que biomatériaux.

La présente invention concerne, également, des polymères susceptibles d'être obtenus à partir des oligomères, tels que décrits ci-dessus et qui répondent à la formule générale suivante :

(II) 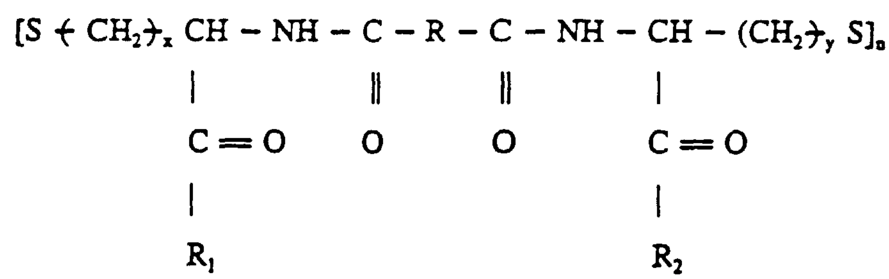

dans laquelle :

- $R_1$ et $R_2$ sont identiques ou différents et sont choisis parmi les groupements suivants :
  - O - $R_5$ ;

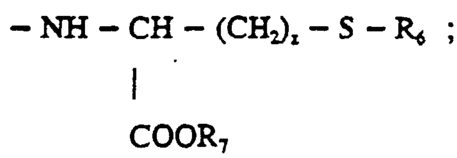

- NH - $CH_2$ - $CH_z$ - S - $R_6$ ; avec $R_5$, $R_6$, $R_7$ représentant, indépendamment, l'hydrogène ou un groupe aliphatique et/ou alicyclique et/ou aromatique, de préference un groupe alkyle inférieur et/ou un groupe aromatique et, plus préférentiellement encore, l'un des groupements suivants :
  - $CH_3$ ; - $CH_2CH_3$ ; - $CH_2$ - Phe ;

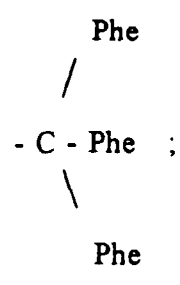

- R est choisi de telle sorte que le radical :

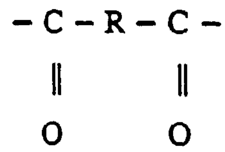

de la formule (I) soit un radical appartenant à la famille des acides poly, avantageusement di, carboxyliques, à l'exclusion de l'acide citrique, R étant, de préférence, sélectionné parmi les groupements suivants :

$(CH_2)_p$ ;

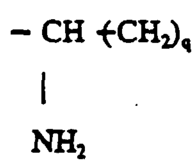 ; 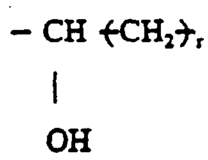

avec :

  - p ≤ 5, de préférence, égal à 2 ou 3,
  - q ≤ 5, de préférence, égal à 1 ou 2
  - et r ≤ 5, de préférence, égal à 1,

- n étant compris entre 1 et 100, de préférence entre 2 et 50 et, plus préférentiellement encore, entre 4 et 30,
- et x et y correspondant à 1 ou 2 comme précédemment.

R peut être, également, composé d'enchaînements polylactique et/ou polyglycolique et/ou polyaminoacide, de faible masse moléculaire.

Ces polymères sont des polysulfures dont l'unité récurrente résulte, de préférence, de l'association d'acide succinique et de cystéine.

Ces polymères peuvent servir de base à l'obtention d'autres produits nouveaux conformes à l'invention et qui sont constitués par des réticulats **(III)**. Cette réticulation s'effectue, par exemple, par amidation et/ou estérification, à l'aide d'au moins un agent de pontage, de préférence choisi parmi les polyols et/ou les polyamides et, plus préférentiellement encore, parmi les produits suivants : cystine, lysine, cystamine et leurs dérivés, oses et leurs dérivés hydrogénés et autres polyols (glycérol).

L'invention concerne, à titre de produits nouveaux, les réticulats **III** réticulés par des ponts issus d'au moins un agent de pontage du type de celui évoqué ci-dessus.

Etant donné que tous les produits conformes à l'invention décrits ci-dessus peuvent s'intégrer dans une même chaîne de préparation, il est clair que la présente invention concerne aussi toute composition constituée par un mélange d'au moins deux des susdits produits.

Les produits selon l'invention sont biocompatibles et se sont révélés être particulièrement appropriés pour entrer dans la composition de biomatériaux.

La présente invention a ainsi également pour objet tout matériau formé d'un mélange et/ou d'une formulation d'au moins l'un des oligomères **(I)** et/ou polymères **(II)**, et/ou réticulats **(III)** et/ou compositions décrits ci-avant avec des macromolécules biologiques, des polymères synthétiques ou naturels biodégradables, tels que :

- les polysaccharides ; e. g. amidon, cellulose, chitosane, dextrane, mucopolysaccharides, tels que l'acide hyaluronique ou chondroïtine sulfate ;
- les protéines ; e. g. collagène, gélatine, albumines, globulines ;
- les polyaminoacides ;
- les polyesters (notamment lactiques et/ou glycoliques), les polyorthoesters, les polyanhydrides, les polyphosphazènes;
- et les lipides et phospholipides.

L'obtention des produits **I**, **II** et **III** selon l'invention s'intègre dans un schéma réactionnel dans lequel la première étape est la préparation de polymères dont, notamment, ceux répondant à la formule **II**, qui donnent accès ensuite aux produits **I**, eux-mêmes retransformables en polymères **II** ou en réticulats **III**.

Selon un mode préféré de mise en oeuvre de l'invention, cette préparation consiste à effectuer :

**a)** - une polycondensation entre :

- d'une part, un réactif de formule A :

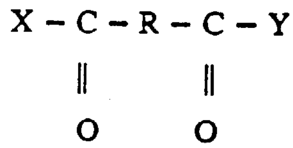

. avec X et Y, identiques ou différents et représentant un halogène, de préférence le chlore ou un radical -$OR_8$, dans lequel $R_8$ correspond à l'hydrogène ou à un radical alicyclique ou aliphatique, de préférence choisi parmi la liste de radicaux suivants :

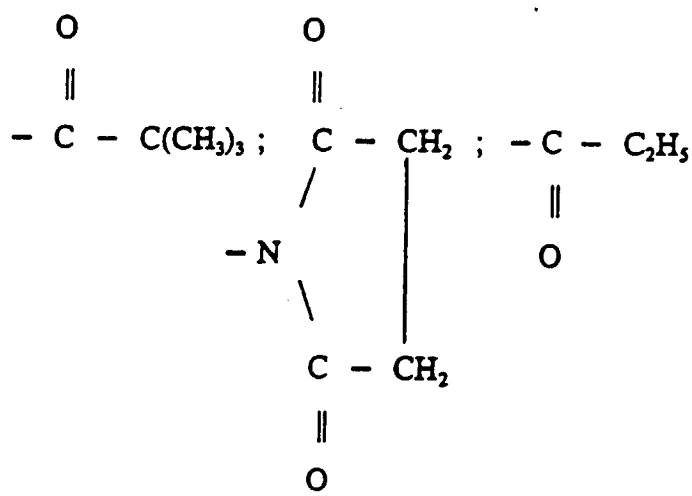

. et avec un radical R qui est une chaîne hydrocarbonée, de préférence alkylée, comportant de 1 à 50 atomes de carbone et, plus préférentiellement encore, aliphatique ayant de 1 à 10 atomes de carbone,

- et, d'autre part, un réactif de formule **B** :

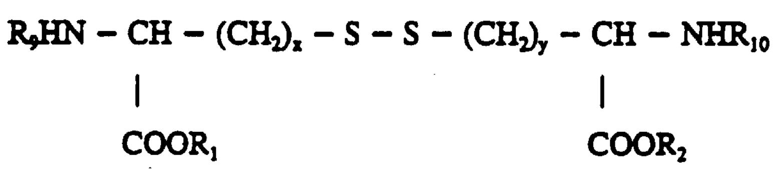

. avec $R_1$ et $R_2$ répondant à une définition identique à celle donnée précédemment,
. avec $R_9$ et $R_{10}$ identiques ou différents et choisis parmi les radicaux suivants : H, aliphatiques, de préférence alkyles, l'hydrogène étant encore celui qui est plus préférentiellement retenu,
. et avec x et étant, classiquement, égal égal 1 ou 2,

**b)** - une réduction du polymère obtenu, ultérieurement transformé ou non.

En pratique, il est préférable que le composé de formule A soit sous forme d'un halogénure d'acide, par exemple

d'un chlorure d'acide, et que le composé **B** de nature cystéique soit estérifié avec des radicaux alkyles $R_1$ et $R_2$ constitués, de préférence, par des radicaux méthyle.

Deux techniques de polycondensation sont envisageables pour obtenir des polymères dont ceux de formule **II** : polycondensation en solution ou polycondensation interfaciale.

Ces techniques seront vues en détail dans les exemples ci-après.

Une fois le polymère obtenu, il est avantageux de procéder à une hydrolyse des fonctions ester portées par ce polymère. Cette hydrolyse est réalisée dans l'eau, en milieu alcalin doux, afin de ménager les fonctions autres que les fonctions esters du polymère (saponification).

Suivant une première variante du procédé conforme à l'invention, le polymère, ayant subi ou non une hydrolyse de ses fonctions ester, est soumis à une réduction b) des ponts disulfure qu'il comporte, ce qui permet d'obtenir majoritairement des oligomères difonctionnels porteurs d'un motif **SH** à chacune de leurs extrémités.

Les techniques de réduction mises en oeuvre sont conventionnelles. Il peut s'agir, par exemple, de celles décrites dans METHODS IN ENZYMOLOGY, vol. 143, "Sulfur and sulfur amino-acids", W. B. JAKOBY, O. W. GRIFFITH, Academic Press Inc., Orlando, (1987).

Suivant une deuxième variante du procédé selon l'invention, on soumet le polymère partiellement ou totalement saponifié à une réticulation. Ce polymère peut être le polycondensat tel quel ou réduit conformément à la première variante du procédé selon l'invention, ce qui correspond aux oligomères difonctionnels di SH. La réticulation s'opère par l'intermédiaire d'au moins un agent de pontage et, de préférence, en présence d'un agent de couplage.

L'agent de pontage est, de préférence, un diol ou une diamine présentant au moins une liaison - S - S -, comme par exemple la cystine dialkyle esther (méthyle ou éthyle).

L'agent de couplage est, avantageusement, choisi dans la liste de composés suivants : éthyl diaminopropyl carbodiimide (EDC), carbonyldiimidazole (CDI).

On peut faire varier le taux de réticulation en jouant sur la quantité d'agent de pontage mis en oeuvre, par rapport au nombre de fonctions acides du polymère.

La concentration en agent de pontage est définie par le ratio suivant :

Suivant l'invention, ce ratio est compris entre 0,01 et 1.

$$\frac{\text{nombre de fonctions } NH_2\text{, OH... de l'agent pontant}}{\text{nombre de fonctions COOH du polymère}}$$

Les réticulats **III** obtenus peuvent être symbolisés comme suit :

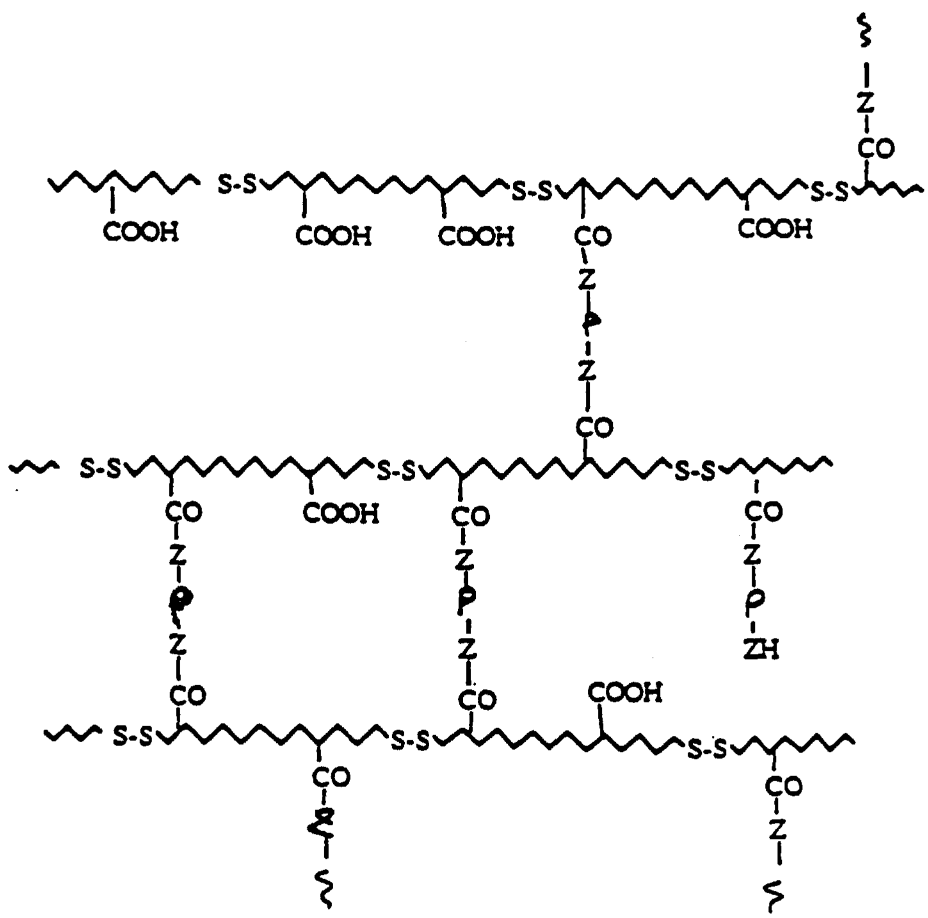

avec Z = O ou NH.

- Z - P - Z - est un pont dérivant des polyols (Z = 0) : HO - P - OH ou des polyamides (Z = NH) : $H_2N$ - P - $NH_2$.

La réduction d'un tel réticulat peut être réalisée en suspension dans l'eau en présence de dithiothréitol ou de tributylphosphine. Elle conduit à un mélange de molécules comportant plusieurs fonctions - **SH** qui peut être isolé, lyophilisé et conservé sous azote à une température inférieure à 0° C. Il est ensuite possible, dans des conditions d'oxydation douces, de reformer les ponts disulfures pour obtenir un réticulat semblable à **(III)**.

Dans le cas particulier où l'agent de pontage est choisi parmi les produits suivants : cystamine ou esters de la cystine ou de l'homocystine, les groupements P du réticulat **(III)** comportent, également, des ponts disulfures et la réduction du réticulat conduit alors à un mélange composé majoritairement des molécules di, tri et tetra-SH décrites plus haut (formule **I**).

La dernière phase du procédé conforme à l'invention, commune aux deux variantes sus-évoquées, consiste à oxyder les oligomères SH obtenus à l'étape précédente, de manière à produire des polymères, dont notamment ceux de formule **(II)**, et/ou des réticulats **(III)**, en (re)formant des ponts disulfures.

Cette oxydation s'effectue, soit et, de préférence, en présence d'au moins un système oxydant comprenant, par exemple, l'iode et/ou ses dérivés et/ou l'eau oxygénée, soit par électrochimie, soit directement à l'air.

La présente invention vise, également, toute composition formée par un mélange d'au moins deux produits de formule **I** et/ou **II** et/ou **III**.

En particulier, les compositions intéressantes sont celles comprenant des mélanges d'oligomères **I**, car une fois reoxydés ceux-ci conduisent à des biomatériaux, gels, revêtements multi-SH, décrits ci-dessus. Ces composés reoxydés doivent présenter un certain nombre de propriétés mécaniques, en relation avec leurs caractéristiques d'usage. Le niveau des propriétés mécaniques est essentiellement dépendant de la structure du réseau formé et de la maîtrise de la réticulation des multi-fonctions, de préférence multi-SH des oligomères. En théorie, toute composition de multi-SH, de fonctionnalité moyenne en SH strictement supérieure à 2, peut donner un réticulat insoluble. La fonctionnalité moyenne en SH peut être définie comme suit :

$$F_{moyenne} = \text{nombre de motif SH par molécule} = \frac{A}{B}$$

avec :

- A = 1. nombre de molécules mono-SH + 2. nombre de molécules di-SH + 3. nombre de molécules tri-SH + 4. nombre de molécules tétra-SH,
- B = nombre de molécules mono-SH + nombre de molécules di-SH + nombre de molécules tri-SH + nombre de molécules tétra-SH.

Compte tenu de la possibilité de réactions intramoléculaires qui perturbent la formation du réseau en consommant des noeuds potentiels, il est préférable de viser des $F_{moyenne}$ pour les mélanges d'oligomères de l'ordre de 2,1 à 2,5, pour assurer la formation du réseau. De façon générale, l'élasticité et le gonflement (aspect gel) du réticulat diminue lorsque la $F_{moyenne}$ augmente.

Une fonctionnalité moyenne souhaitée (par exemple 2,3) peut être obtenue directement ou indirectement.

Selon la méthode directe, on réticule les polycondensats linéaires de longueur connue, afin d'estimer la proportion relative de mono-SH par rapport aux di-SH, avec une quantité adaptée d'agent pontant, tel que la cystine diméthylester. Après réduction du réticulat obtenu, on dispose d'un mélange de mono, di, tri, tetra-SH dont la $F_{moyenne}$ sera proche de celle désirée. Il faut s'assurer, toutefois, de la réactivité totale de l'agent pontant et de la totalité de la réduction des ponts SS.

La méthode indirecte consiste à "sur-réticuler" un polymère linéaire en visant une $F_{moyenne}$ théorique, voisine de 3 par exemple, de réduire ce réticulat, de déterminer par dosage la $F_{moyenne}$ obtenue, de préparer, par réduction d'un polycondensat linéaire, un mélange de mono et de di-SH proche de 2 et d'obtenir, en mélangeant les deux compositions dosées dans des proportions voulues, pour obtenir la $F_{moyenne}$ correspondant à un optimum des propriétés recherchées.

Pour les applications de biomatériaux nécessitant la formation d'un gel, il paraît souhaitable de partir d'une composition, i. e. d'un mélange d'oligomères ayant une $F_{moyenne}$ supérieure ou égale à 2, de préférence inférieure ou égale à 2,6 et, plus préférentiellement encore, à 2,3.

Pour des objets plus durs, ciments de comblement, pièces d'ostéosynthèse, implants rigides, il paraît souhaitable de viser des $F_{moyennes}$ supérieures ou égales à 2,3 et, préférentiellement, à 2,5.

Les oligomères **(I)**, polymères **(II)** entre autres, réticulats **(III)**, fonctionnalisés ou non, conformes à l'invention sont des composés ne présentant aucune toxicité directe ou indirecte : ils ne sont pas carcinogènes, tératogènes, immunogènes ni mutagènes. Par ailleurs, ils sont parfaitement biodégradables, c'est-à-dire qu'ils sont constitués de produits s'intégrant parfaitement bien dans les voies métaboliques (cycle Krebs notamment) de l'être humain ou animal. Les produits de dégradation de ces composés sont ipso facto parfaitement tolérés.

**POSSIBILITE D'APPLICATION INDUSTRIELLE :**

Les produits conformes à l'invention trouvent des applications intéressantes à titre de biomatériau, pouvant, par exemple, servir de base à la fabrication de sutures, de ligatures, de prothèses chirurgicales vasculaires, osseuses, tissulaires ou ligamentaires, d'implants de toutes natures ou bien encore de système de libération contrôlée de principes actifs.

En particulier, il est intéressant de noter que les oligomères **(I)** selon l'invention ont une faible masse molaire (inférieure à 1 000 Da) et qu'ils sont ainsi capables de diffuser à l'intérieur des tissus biologiques pour y être ensuite polymérisés et/ou réticulés. Les enchevêtrements qu'ils forment alors avec les glyco-protéines assurent un lien adhésif solide.

Sous forme réduite et associés à un système oxydant, ces produits et/ou leurs mélanges conviennent très bien à titre de constituants de ciments chirurgicaux ou dentaires, d'agents de comblement ou de colles biologiques. Ces constituants rentrent dans le champ de l'invention.

Sous forme oxydée, ces constituants sont des réseaux cohésifs, parsemés de ponts disulfures et ayant entre eux des propriétés mécaniques et biologiques modulables.

Les exemples 1 à 10 qui suivent sont une illustration des propriétés et des variantes structurelles des produits selon l'invention. Ils décrivent, également, les structures et les méthodes de préparation des produits selon l'invention.

## EXEMPLES

**EXEMPLE 1** : SYNTHÈSE DU POLYMÈRE ① PAR POLYCONDENSATION EN SOLUTION DANS LE DIMÉTHYLACÉTAMIDE (DMAC) DU CHLORHYDRATE DE LA CYSTINE DIMÉTHYLESTER ET DU CHLORURE DE SUCCINYLE.

①

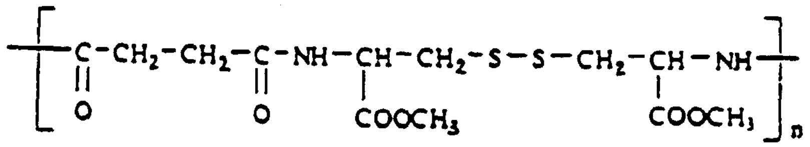

25 g (0,073 mol) de chlorhydrate de cystine diméthylester et 400 ml de DMAC sont placés dans un réacteur de 1 l. 41,2 ml de triéthylamine (0,293 mol) sont alors ajoutés. 8,1 ml de chlorure de succinyle fraîchement distillé sont dilués dans 100 ml de DMAC, le tout est ajouté au mélange réactionnel à l'aide d'une ampoule de coulée. Le mélange réactionnel est alors agité pendant 24 heures à température ambiante. Le sel de triéthylammonium précipité est enlevé par filtration, puis le mélange réactionnel est précipité dans 5 l d'eau. Le polymère est récupéré par filtration, séché à l'étuve sous vide : 13 g d'une poudre blanche (légèrement rosée) sont ainsi obtenus. Les spectres RMN $^1H$ (dans l'acide trifluoroacétique deutéré (TFA) et IR sont conformes. Les masses molaires, déterminées par chromatographie d'exclusion stérique (SEC) dans le DMAC et exprimées en équivalents polystyrène, sont les suivantes :

$$M_n = 6\,200,\ M_w = 9\,600$$

**EXEMPLE 2** : SUNTHÈSE DU POLYMÈRE ① PAR POLYCONDENSATION INTERFACIALE EAU/TOLUÈNE DU CHLORHYDRATE DE LA CYSTINE DIMÉTHYLESTER ET DU CHLORURE DE SUCCINYLE.

25 g (0,073 mol) de chlorhydrate de cystine diméthylester et 200 ml de DMAC sont placés dans un réacteur de 11. 31,06 g de carbonate de sodium anhydre (0,293 mol) sont alors ajoutés. Une préémulsion est ensuite formée par addition de 100 ml de toluène. 8,1 ml de chlorure de succinyle fraîchement distillé sont alors dilués dans 100 ml de toluène, le tout est ajouté au mélange réactionnel à l'aide d'une ampoule de coulée. Le mélange réactionnel est alors agité pendant 4 heures à température ambiante. Le polymère, précipité au cours de la réaction, est récupéré par filtration, lavé avec de l'acétone, puis avec de l'eau. Il est séché à l'étuve sous vide : 14 g d'ne poudre blanche (légèrement rosée) sont ainsi obtenus. Les spectres RMN 'H (dans le TFA) et IR sont conformes et analogues à ceux obtenus pour le polymère de l'**exemple 1**. Les masses molaires, déterminées par SEC dans le DMAC et exprimées en équivalents polystyrène, sont les suivantes :

$$M_n = 5\,700,\ M_w = 11\,500$$

**EXEMPLE 3** : HYDROLYSE DES FONCTIONS ESTER DU POLYMÈRE ①: OBTENTION DU POLYMÈRE ②

②

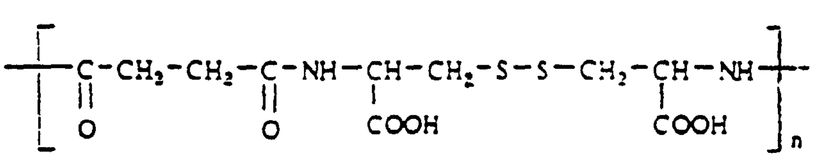

5 g de polymère ① obtenu par polycondensation en solution ou interfaciale sont mis en suspension dans 1 l d'eau. Le pH est ajusté à 10.5 avec de la soude 1 M et maintenu à cette valeur pendant toute la durée de l'hydrolyse. L'addition de soude est arrêtée lorsque la solution est devenue limpide. La solution est alors acidifiée jusqu'à un pH $< 3$ par une résine échangeuse d'ion acide. Elle est concentrée, congelée, puis lyophilisée. 4,6 g d'une poudre blanche sont obtenus. Les spectres RMN $^1H$ (dans le TFA et dans $D_2O$) et IR sont conformes et montrent que l'hydrolyse des fonctions esters est totale.

**EXEMPLE 4 :** **RÉDUCTION DU POLYMÈRE ② PAR LE DITHIOTHRÉITOL : OBTENTION DE LA MOLÉCULE ③**

③ 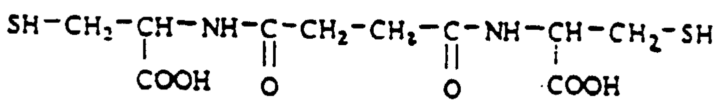

3 g de polymère ② et 2,87 g de dithiothréitol (DTT) sont dissous dans 70 ml d'eau sous atmosphère d'azote. Le pH est ajusté à 8,5 par addition de soude 1 M et la solution est agitée pendant 3 heures sous bulle à bulle d'azote. Le mélange est alors extrait deux fois par 100 ml d'acétate d'éthyle. La phase aqueuse est ensuite acidifiée par une résine échangeuse d'ions acide jusqu'à pH = 4,5, puis concentrée et précipitée dans un excès d'acétone. Le précipité collant obtenu est redissous dans un minimum d'eau et reprécipité dans l'acétone. Il est finalement redissous dans l'eau et lyophilisé. 2 g d'un produit légèrement jaune sont récupérés. Le spectre RMN $^1H$ (dans $D_2O$) obtenu est conforme à la formule ③, les groupements carboxyliques étant sous forme ionisée.

**EXEMPLE 5** : RÉDUCTION DU POLYMÈRE ② PAR LA TRI(N-BUTYL)PHOSPHINE : OBTENTION DE LA MOLÉCULE ③.

2,4 g de polymère ② sont dissous dans 30 ml d'eau sous atmosphère d'azote. 120 ml de méthanol, préalablement dégazé, sont alors ajoutés. Puis, 2 ml de tri (n-butyl) phosphine sont injectés dans le mélange réactionnel. Après 3 heures de réaction, le méthanol est évaporé à l'aide d'un évaporateur rotatif. 50 ml d'eau sont rajoutés à la solution aqueuse résiduelle qui est ensuite extraite deux fois avec 200 ml d'acétate d'éthyle. La solution aqueuse est ensuite acidifiée et précipitée dans l'acétone, comme décrit dans l'**exemple 4**. Le spectre RMN $^1H$ dans $D_2O$ est identique à celui du produit obtenu dans l'**exemple 4**.

**EXEMPLE 6** : RÉTICULATION DU POLYMÈRE ② PAR LA CYSTINE DIMÉTHYLESTER.

5 g du polymère ② et 5,3 g de chlorhydrate de cystine diméthylester sont dissous dans 100 ml d'eau. 6 g de N-diméthylaminopropyl, N'-éthyl carbodiimide (EDC) sont alors dissous dans 5 ml d'eau et aussitôt rajoutés dans le mélange réactionnel. Le mélange prend immédiatement une coloration rouge foncé puis, au bout de quelques secondes, il se forme un précipité rose. La réaction est arrêtée après 3 heures et 200 ml d'eau sont rajoutés. Le précipité est récupéré par filtration, lavé plusieurs fois avec de l'eau, puis séché à l'étuve sous vide.

**EXEMPLE 7** : RÉTICULATION DU POLYMÈRE ② PAR LA CYSTINE DIÉTHYLESTER.

5 g du polymère ② et 5,73 g de chlorhydrate de cystine diéthylester sont dissous dans 100 ml d'eau. 6 g de N-diméthylaminopropyl, N'-éthyl carbodiimide (EDC) sont alors dissous dans 5 ml d'eau et aussitôt rajoutés dans le mélange réactionnel. La réaction est arrêtée après 3 heures et 200 ml d'eau sont rajoutés. Le précipité est récupéré par filtration, lavé plusieurs fois avec de l'eau puis séché à l'étuve sous vide.

**EXEMPLE 8** : RÉDUCTION PAR LE DITHIOTHRÉITOL DU POLYMÈRE RÉTICULÉ DE L'EXEMPLE 6.

1 g du polymère réticulé de l'**exemple 7** et 1,1 g de dithiothréitol sont dissous dans 50 ml d'eau, préalablement purgée par un courant d'azote. Le pH est ajusté à 9,5 par la soude 1 M. Le mélange réactionnel devient limpide et la réaction est arrêtée au bout d'une heure. Après six extractions avec 50 ml d'acétate d'éthyle, la solution aqueuse est acidifiée jusqu'à pH = 5 par une résine échangeuse, réextraite avec deux fois 50 ml d'acétate d'éthyle puis lyophilisée. Le produit obtenu est un mélange comprenant, principalement, les molécules ③, ④, ⑤ suivantes :

③ 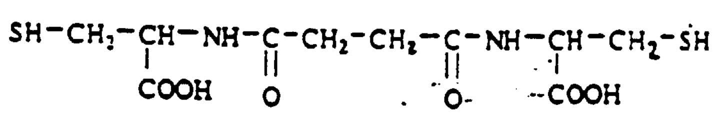

④

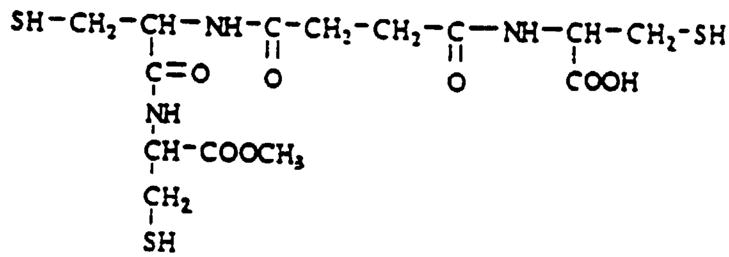

⑤

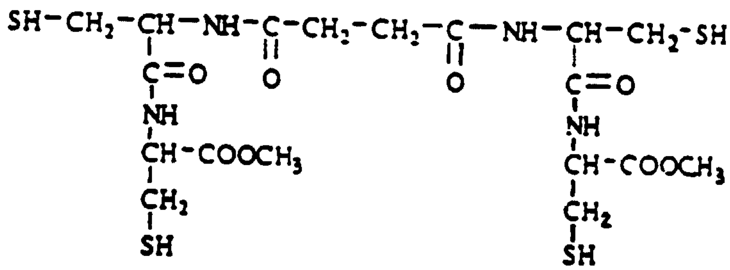

Les fonctions carboxyliques sont sous forme ionisée (- $COO^-$, $Na^+$). Le mélange n'est plus entièrement soluble dans l'eau lorsque le pH est <3.

**EXEMPLE 9** : RÉDUCTION PAR LE DITHIOTHRÉITOL DU POLYMÈRE RÉTICULÉ DE L'EXEMPLE 6 - HYDROLYSE DES FONCTIONS ESTER DU PRODUIT OBTENU.

La réaction est réalisée, comme décrit à l'**exemple 8**, mais la solution réduite est maintenue à pH = 9,5 pendant 24 heures à 35° C. Après six extractions avec 50 ml d'acétate d'éthyle, la solution aqueuse est acidifiée jusqu'à pH = 5 par une résine échangeuse, réextraite avec deux fois 50 ml d'acétate d'éthyle, puis lyophilisée. Le produit obtenu est un mélange comprenant, principalement, les molécules ③, ⑥, ⑦ suivantes :

③

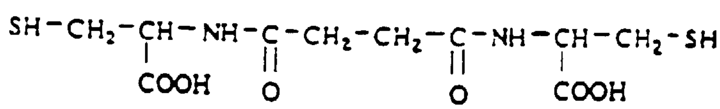

⑥

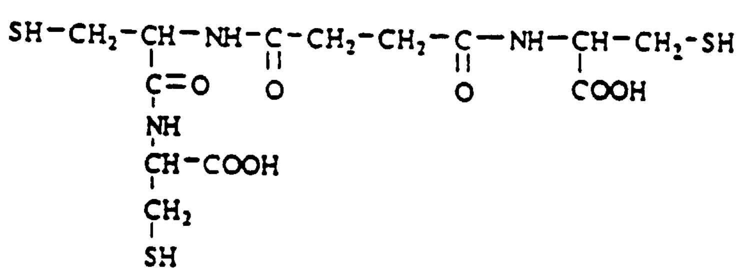

⑦ 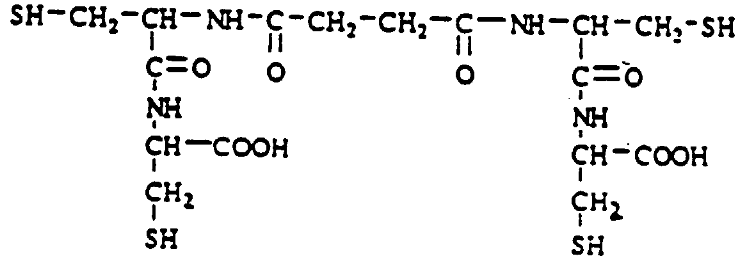

Les fonctions carboxyliques sont sous forme ionisée (- $COO^-$, $Na^+$). Le mélange peut être acidifié (jusqu'à pH = 2,5) par passage sur une résine échangeuse d'ions. Dans ce cas, la solubilité dans l'eau est conservée.

**EXEMPLE 10** : RÉDUCTION PAR LE DITHIOTHRÉITOL DU POLYMÈRE RÉTICULÉ DE L'EXEMPLE 7.

1 g du polymère réticulé de l'**exemple 7** et 1,1 g de dithiothréitol sont dissous dans 50 ml d'eau, préalablement purgée par un courant d'azote. Le pH est ajusté à 9,5 par de la soude 1 M. Le mélange réactionnel devient limpide et la réaction est arrêtée au bout d'une heure. Après six extractions avec 50 ml d'acétate d'éthyle, la solution aqueuse est acidifiée jusqu'à pH = 4 par une solution HCl 1N. Un précipité collant, légèrement marron est obtenu. Il s'agit d'un mélange constitué, principalement, des molécules ③, ⑧, ⑨ suivantes :

③ 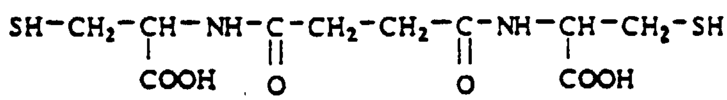

⑧ 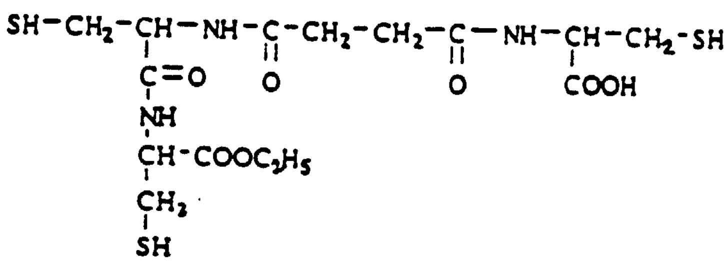

⑨ 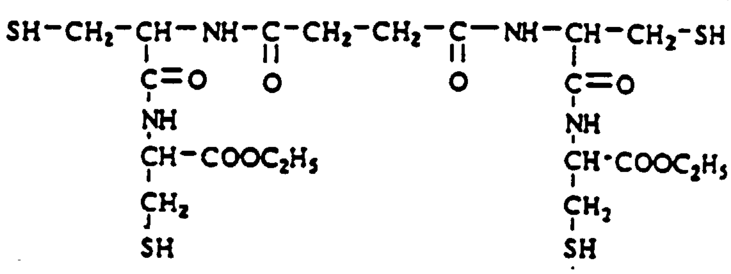

## Revendications

**1.** Nouveau produit organique contenant au moins deux fonctions thiols ou dérivés et des fonctions carboxyliques, protégées ou non, et/ou des fonctions carbonylées, de formule générale suivante :

(I) 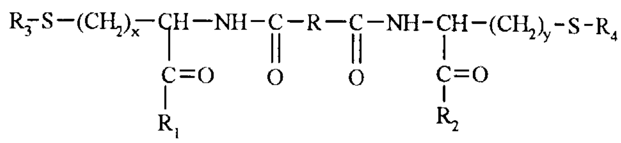

dans laquelle :

- R est une chaîne hydrocarbonée éventuellement substituée, de préférence alkylée comportant de 1 à 50 atomes de carbone et, plus préférentiellement encore, aliphatique, ayant de 1 à 10 atomes de carbone,
- $R_1$ et $R_2$ sont identiques ou différents et sont choisis parmi les groupements suivants :
  - O - $R_5$ ;

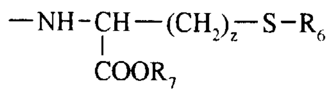 ;

$$-NH-(CH_2)_y-S-R_6$$

- $R_3$, $R_4$, $R_5$, $R_6$ et $R_7$ représentent, indépendamment, l'hydrogène ou un groupe aliphatique et/ou alicyclique et/ou aromatique, de préférence un groupe alkyle comprenant de 1 à 6 atomes de carbone et/ou un groupe aromatique et, plus préférentiellement encore, l'un des groupements suivants :
  - $CH_3$ ; - $CH_2CH_3$ ;

$$-CH_2-Phe$$ ;

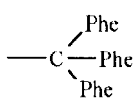

- x, y, z = 1 ou 2,

**à l'exclusion** des produits de formule (I) dans lesquels :

- R = $-(CH_2)_t$ avec t = 2,4 ou 6 à 12 ou phénylène,
- $R_1$ et $R_2$ sont identiques et correspondent à OH, - O - alkyle ou - N - alkyle,
- $R_3$ et $R_4$ sont identiques et représentent l'hydrogène, - $CH_3$ ou $-CH_2$ - COOH,
- et x et y sont identiques et sont égaux à 1 ou 2.

**2.** Produit selon la revendication 1, caractérisé en ce que $R_1$ et $R_2$ représentent - O - $R_5$ .

**3.** Produit selon la revendication 1, caractérisé en ce que $R_1$ représente :

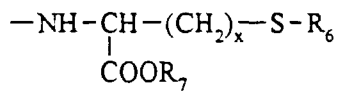

et $R_2$ représente - O - $R_5$ ou inversement.

**4.** Produit selon la revendication 1, caractérisé en ce que $R_1$ et $R_2$ sont constitués par le radical :

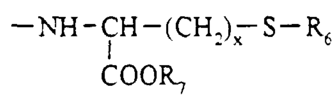

**5.** Produit selon l'une quelconque des revendications 1 à 4, caractérisé en ce qu'il permet d'obtenir, après oxydation, des polymères, réticulés ou non, utilisables comme biomatériaux et, éventuellement, dégradables en métabolites naturels.

**6.** Produit selon l'une quelconque des revendications 1 à 5, caractérisé en ce que R est choisi de telle sorte que le radical :

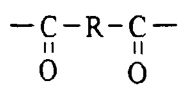

de la formule **(I)** soit un radical appartenant à la famille des restes d'acides poly, avantageusement di-carboxyliques, à l'exclusion de l'acide citrique, R étant, de préférence, sélectionné parmi les groupements suivants :

$-(CH_2)_p-$ ; 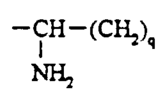 ; 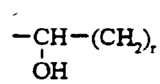

avec :

- $p \leq 5$, de préférence, égal à 3 ,
- $q \leq 5$, de préférence, égal à 1 ou à 2,
- et enfin, $r \leq 5$, de préférence, égal à 1.

**7.** Produit selon l'une quelconque des revendications 1 à 5, caractérisé en ce que R est composé d'enchaînements polylactique et/ou polyglycolique et/ou polyaminoacide, de faible masse moléculaire.

**8.** Polymères, susceptibles d'être obtenus à partir des produits selon la revendication 6 ou la revendication 7, caractérisés en ce qu'ils répondent à la formule générale suivante :

**(II)** 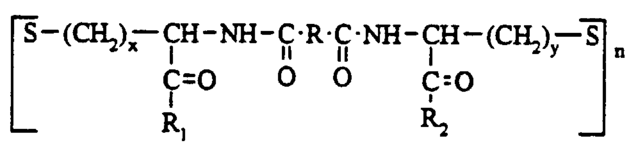

n est compris entre 1 et 100 et dans laquelle $R_1$ et $R_2$ sont tels que définis ci-dessus selon l'une quelconque des revendications 1 à 4 et R est tel que défini à la revendication 6 ou la revendication 7.

**9.** Réticulats, caractérisés en ce qu'ils sont constitués par des polymères selon la revendication 8, réticulés par des ponts issus d'au moins un agent de pontage, de préférence choisi parmi les polyols et/ou les polyamines et, plus préférentiellement encore, parmi les produits suivants : cystine et ses dérivés, lysine et ses dérivés, oses et leurs dérivés hydrogénés, autres polyols (glycérol).

**10.** Composition, caractérisée en ce qu'elle est constituée par un mélange d'au moins deux des produits selon l'une quelconque des revendications 1 à 9.

**11.** Composition selon la revendication 10, caractérisée en ce qu'elle est formée par un mélange d'oligomères de formule (I) et en ce qu'elle présente une $F_{moyenne}$ supérieure ou égale à 2.

**12.** Procédé de préparation d'un produit selon l'une quelconque des revendications 1 à 7, caractérisé en ce qu'il consiste, essentiellement, à mettre en oeuvre :

a) - une polycondensation entre :

- un réactif de formule **A** :

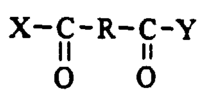

. avec X et Y, identiques ou différents et représentant un halogène, de préférence le chlore ou un radical - $OR_8$, dans lequel $R_8$ correspond à l'hydrogène ou à un radical aliphatique et/ou alicyclique,
. et avec R conforme à la définition donnée dans la revendication 1,

- et un réactif de formule **B** :

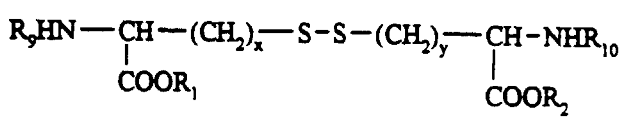

. avec $R_1$ et $R_2$ tels que définis dans la revendication 1,
. avec $R_9$ et $R_{10}$ identiques ou différents et choisis parmi les radicaux suivants: H, aliphatiques, tels que les alkyles, l'hydrogène étant particulièrement préféré,
. et avec x et y = 1 ou 2,

**b)** - une réduction du polymère obtenu, ultérieurement transformé ou non.

**13.** Procédé selon la revendication 12, caractérisé en ce que l'on effectue une hydrolyse des fonctions esters du polymère obtenu à l'issue de l'étape **a)**.

**14.** Procédé selon la revendication 12 ou 13, caractérisé en ce que, préalablement à l'étape **b)**, on soumet le polymère au moins partiellement saponifié à une réticulation s'opérant par l'intermédiaire d'au moins un agent de pontage et, de préférence, en présence d'un agent de couplage, de manière à obtenir le réticulat du type de ceux selon la revendication 9.

**15.** Procédé selon la revendication 14, caractérisé en ce que les produits selon au moins l'une des revendications 1 à 6 sont hydrolysés de manière à ce que $R_1$, $R_2$ = OH.

**16.** Procédé de préparation des polymères selon la revendication 8 et/ou des réticulats selon la revendication 9, caractérisé en ce que l'on oxyde les produits obtenus à partir du procédé selon l'une quelconque des revendications 12 à 15, de préférence à l'aide d'un système oxydant constitué, de préférence, par de l'iode et/ou ses dérivés et/ou par de l'eau oxygénée.

**17.** Biomatériau, caractérisé en ce qu'il est constitué par au moins l'un des produits, polymères, réticulats et compositions, selon l'une au moins des revendications 1 à 11.

**18.** Biomatériau, caractérisé en ce qu'il est constitué par au moins l'un des produits, polymères, réticulats et compositions, selon l'une au moins des revendications 1 à 11 et par un système oxydant.

**19.** Biomatériau, caractérisé en ce qu'il est constitué par un mélange et/ou une formulation d'au moins l'un des produits et/ou polymères et/ou réticulats, et/ou compositions selon au moins l'une des revendications 1 à 11 avec des macromolécules biologiques ou des polymères synthétiques ou naturels biodégradables, tels que :

- les polysaccharides ; e. g. amidon, cellulose, chitosane, dextrane, mucopolysaccharides, tels que l'acide hyaluronique ou chondroitine sulfate ;
- les protéines ; e. g. collagène, gélatine, albumine, globulines ;
- les polyaminoacides ;
- les polyesters (notamment lactiques et/ou glycoliques), les polyorthoesters, les polyanhydrides, les polyphosphazènes ;
- et les lipides et phospholipides.

### Patentansprüche

**1.** Neues organisches Produkt, das mindestens zwei Thiolgruppen oder Derivate von Thiolgruppen und geschützte oder nicht geschützte Carboxygruppen und/oder Carbonylgruppen enthält, der folgenden allgemeinen Formel:

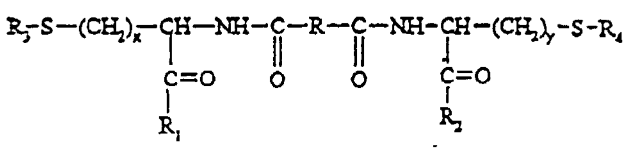

(I)

in der bedeuten:

- R eine gegebenenfalls substituierte Kohlenwasserstoffkette, bei der es sich vorzugsweise um eine Alkylkette mit 1 bis 50 Kohlenstoffatomen und noch bevorzugter eine aliphatische Kette mit 1 bis 10 Kohlenstoffatomen handelt,
- $R_1$ und $R_2$ Gruppen, die gleich oder verschieden sind und unter den folgenden Gruppen

  $- O - R_5$,

  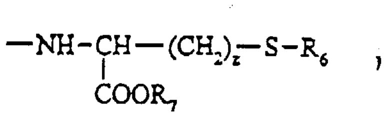

  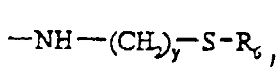

  ausgewählt sind,
- $R_3$, $R_4$, $R_5$, $R_6$ und $R_7$ unabhängig Wasserstoff oder eine aliphatische und/oder alicyclische und/oder aromatische Gruppe, vorzugsweise eine Alkylgruppe mit 1 bis 6 Kohlenstoffatomen und/oder eine aromatische Gruppe und noch bevorzugter eine der folgenden Gruppen:

  $-CH_3$, $-CH_2-CH_3$, $-CH_2-Phe$

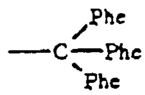

- x, y, z = 1 oder 2,

wobei Produkte der Formel (I) ausgeschlossen sind, in der bedeuten:

- R = $-(CH_2)_t$ mit t = 2,4 oder 6 bis 10, oder Phenylen,
- $R_1$ und $R_2$ Gruppen, die identisch sind und -OH, -O-Alkyl oder -N-Alkyl entsprechen,
- $R_3$ und $R_4$ Gruppen, die identisch sind und Wasserstoff, $-CH_3$ oder $-CH_2$-COOH bedeuten,
- und x und y Zahlen, die identisch sind und die gleich 1 oder 2 sind.

**2.** Produkt nach Anspruch 1, dadurch gekennzeichnet, daß $R_1$ und $R_2$ die Gruppe -O-$R_5$ darstellen.

**3.** Produkt nach Anspruch 1, dadurch gekennzeichnet, daß $R_1$

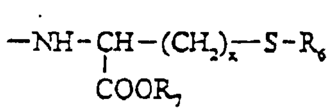

und $R_2$ -O-$R_5$ bedeutet oder umgekehrt.

**4.** Produkt nach Anspruch 1, dadurch gekennzeichnet, daß $R_1$ und $R_2$ aus der Gruppe

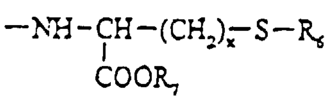

bestehen.

**5.** Produkt nach einem der Ansprüche 1 bis 4, dadurch gekennzeichnet, daß mit ihm nach einer Oxidation vernetzte oder nicht vernetzte Polymere erhalten werden können, die als Biomaterialien verwendbar sind und gegebenenfalls zu natürlichen Metaboliten abbaubar sind.

**6.** Produkt nach einem der Ansprüche 1 bis 5, dadurch gekennzeichnet, daß R so ausgewählt ist, daß die Gruppe

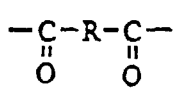

der Formel (I) eine Gruppe ist, die zur Familie der Polycarbonsäurereste, vorzugsweise der Dicarbonsäurereste, gehört, wobei Citronensäure ausgeschlossen ist, wobei R vorzugsweise unter den folgenden Gruppen

$-(CH_2)_p-$,

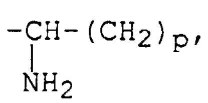

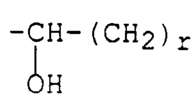

ausgewählt ist, in denen bedeuten:

- $p \leq 5$, vorzugsweise gleich 3,
- $q \leq 5$, vorzugsweise gleich 1 oder 2,
- und schließlich $r \leq 5$, vorzugsweise gleich 1.

**7.** Produkt nach einem der Ansprüche 1 bis 5, dadurch gekennzeichnet, daß R aus Polymilchsäure- und/oder Polyglykolsäure- und/oder Polyaminosäure-Kette mit geringer Molmasse besteht.

**8.** Polymere, die aus Produkten nach Anspruch 6 oder Anspruch 7 hergestellt werden können, dadurch gekennzeichnet, daß sie die folgende allgemeine Formel:

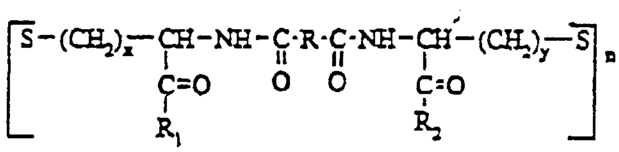 (II)

aufweisen, in der n im Bereich von 1 bis 100 liegt und in der $R_1$ und $R_2$ wie weiter oben in einem der Ansprüche 1 bis 4 definiert sind und R so wie in Anspruch 6 oder Anspruch 7 definiert ist.

**9.** Vernetzte Produkte, dadurch gekennzeichnet, daß sie aus Polymeren nach Anspruch 8 bestehen, die über Brücken vernetzt sind, die von mindestens einem Mittel zur Brückenbildung stammen, das vorzugsweise unter mehrwertigen Alkoholen und/oder Polyaminen und noch bevorzugter unter Cystin und dessen Derivaten, Lysin und dessen Derivaten, Zuckern und deren hydrierten Derivaten, sonstigen mehrwertigen Alkoholen (Glycerin) ausgewählt ist.

**10.** Zusammensetzung, dadurch gekennzeichnet, daß sie aus einem Gemisch von mindestens zwei Produkten nach einem der Ansprüche 1 bis 9 besteht.

**11.** Zusammensetzung nach Anspruch 10, dadurch gekennzeichnet, daß sie aus einem Gemisch von Oligomeren der Formel (I) gebildet ist und daß sie eine mittlere Funktionalität $F_{mittel}$ größer als oder gleich 2 aufweist.

**12.** Verfahren zur Herstellung eines Produkts nach einem der Ansprüche 1 bis 7, dadurch gekennzeichnet, daß es im wesentlichen darin besteht, die folgenden Schritte durchzuführen:

a) Polykondensation

- eines Reaktionspartners der Formel A:

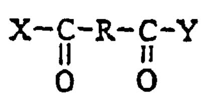

  . in der X und Y, die gleich oder verschieden sind, die ein Halogen, vorzugsweise Chlor, oder eine Gruppe $-OR_8$ darstellen, worin $R_8$ Wasserstoff oder eine aliphatische und/oder alicyclische Gruppe bedeutet,
  . und in der R wie in Anspruch 1 definiert ist,

- mit einem Reaktionspartner der Formel B:

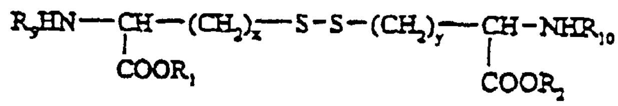

- in der $R_1$ und $R_2$ wie in Anspruch 1 definiert sind,
- in der $R_9$ und $R_{10}$, die gleich oder verschieden sind, unter Wasserstoff, aliphatischen Gruppen, wie z.B. Alkylgruppen, ausgewählt sind, wobei Wasserstoff besonders bevorzugt ist,
- und in der x und y gleich 1 oder 2 sind,

b) Reduktion des erhaltenen und anschließend gegebenenfalls umgewandelten Polymers.

**13.** Verfahren nach Anspruch 12, dadurch gekennzeichnet, daß eine Hydrolyse der Estergruppen des am Ende von Stufe a) erhaltenen Polymers durchgeführt wird.

**14.** Verfahren nach Anspruch 12 oder 13, dadurch gekennzeichnet, daß vor Durchführung von Stufe b) das mindestens teilweise verseifte Polymer einer Vernetzung unterzogen wird, die mit Hilfe mindestens eines Mittels zur Brückenbildung und vorzugsweise in Gegenwart eines Kupplungsmittels durchgeführt wird, um das vernetzte Produkt gemäß Anspruch 9 zu erhalten.

**15.** Verfahren nach Anspruch 14, dadurch gekennzeichnet, daß die Produkte nach mindestens einem der Ansprüche 1 bis 6 hydrolysiert werden, damit $R_1$ und $R_2$ Hydroxygruppen sind.

**16.** Verfahren zur Herstellung von Polymeren nach Anspruch 8 und/oder vernetzten Produkten nach Anspruch 9, dadurch gekennzeichnet, daß die bei dem Verfahren nach einem der Ansprüche 12 bis 15 erhaltenen Produkte oxidiert werden, vorzugsweise mit einem oxidierenden System, das vorzugsweise aus Iod und/oder dessen Derivaten und/oder Wasserstoffperoxid besteht.

**17.** Biomaterial, dadurch gekennzeichnet, daß es aus mindestens einer der Produkte, Polymere, vernetzten Produkte und Zusammensetzungen nach mindestens einem der Ansprüche 1 bis 11 besteht.

**18.** Biomaterial, dadurch gekennzeichnet, daß es aus mindestens einem der Produkte, Polymere, vernetzten Produkte und Zusammensetzungen nach mindestens einem der Ansprüche 1 bis 11 und einem oxidierenden System besteht.

**19.** Biomaterial, dadurch gekennzeichnet, daß es aus einem Gemisch und/oder einer Formulierung aus mindestens einem der Produkte und/oder Polymere und/oder vernetzten Produkte und/oder Zusammensetzungen nach mindestens einem der Ansprüche 1 bis 11 und biologischen Makromolekülen oder biologisch abbaubaren, synthetischen oder natürlichen Polymeren, wie z.B.

- Polysacchariden, z.B. Stärke, Cellulose, Chitosan, Dextran, Mucopolysacchariden, wie z.B. Hyaluronsäure oder Chondroitinsulfat,
- Proteinen, z.B. Collagen, Gelatine, Albumin, Globulinen,
- Polyaminosäuren,
- Polyestern (insbesondere Milchsäure- und/oder Glykolsäureestern), Polyorthoestern, Polyanhydriden, Polyphosphazenen,
- und Lipiden und Phospholipiden

besteht.

**Claims**

**1.** Novel organic product containing at least two thiol or derived functions and carboxyl functions, which may or may not be protected, and/or carbonyl functions, of following general formula :

**(I)** 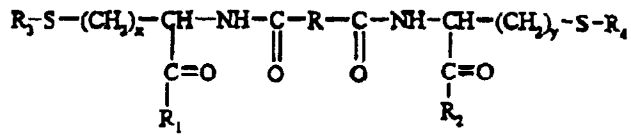

in which :

- R is an optionally substituted hydrocarbon chain, preferably an alkyl chain containing from 1 to 50 carbon atoms and, more preferentially still, an aliphatic chain having from 1 to 10 carbon atoms,
- $R_1$ and $R_2$ are identical or different and are chosen from the following groups :
  $-O-R_5$ ;

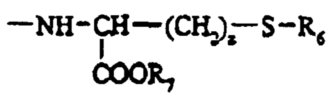

;

$-NH-(CH_2)_y-S-R_6$

- $R_3$, $R_4$, $R_5$, $R_6$ and $R_7$ independently represent hydrogen or an aliphatic and/or alicyclic and/or aromatic group, preferably an alkyl group comprising from 1 to 6 carbon atoms and/or an aromatic group and, more preferentially still, one of the following groups :
  $-CH_3$ ; $-CH_2CH_3$ ;

$-CH_2-Phe$ ;

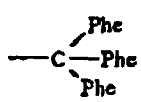

- x, y and z = 1 or 2,

**with the exception** of the products of formula **(I)** in which :

- R = $-(CH_2)_t$ with t = 2, 4 or 6 to 12 or phenylene,
- $R_1$ and $R_2$ are identical and correspond to OH, -O-alkyl or -N-alkyl,
- $R_3$ and $R_4$ are identical and represent hydrogen, $-CH_3$ or $-CH_2-COOH$,
- and x and y are identical and are equal to 1 or 2.

**2.** Product according to Claim 1, characterized in that $R_1$ and $R_2$ represent $-O-R_5$.

**3.** Product according to Claim 1, characterized in that $R_1$ represents :

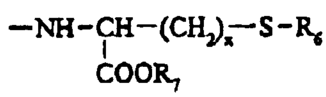

and $R_2$ represents $-O-R_5$, or vice versa.

**4.** Product according to Claim 1, characterized in that $R_1$ and $R_2$ consist of the radical :

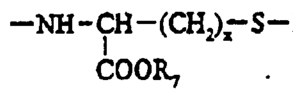

**5.** Product according to any one of Claims 1 to 4, characterized in that it makes it possible to obtain, after oxidation, polymers, which may or may not be crosslinked, which can be used as biomaterials and, optionally, which can be degraded to natural metabolites.

**6.** Product according to any one of Claims 1 to 5, characterized in that R is chosen so that the radical :

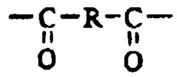

of the formula **(I)** is a radical belonging to the group of the residues of poly-, advantageously di-, carboxylic acids, with the exception of citric acid, R preferably being selected from the following groups :

$-(CH_2)_p-$ ; 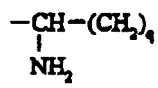 ; 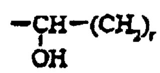

with :

- $p \leq 5$, preferably equal to 3,
- $q \leq 5$, preferably equal to 1 or to 2,
- and, finally, $r \leq 5$, preferably equal to 1.

**7.** Product according to any one of Claims 1 to 5, characterized in that R is composed of low molecular mass polylactic and/or polyglycolic and/or poly(amino acid) chains.

**8.** Polymers capable of being obtained from the products according to Claim 6 or Claim 7, characterized in that they correspond to the following general formula :

**(II)** 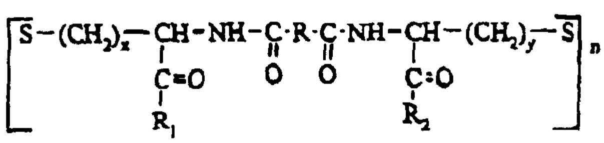

n is between 1 and 100 and in which $R_1$ and $R_2$ are as defined above according to any one of Claims 1 to 4 and R is as defined in Claim 6 or Claim 7.

**9.** Networks, characterized in that they are composed of polymers according to Claim 8 crosslinked by bridges resulting from at least one bridging agent, preferably chosen from polyols and/or polyamines and, more preferentially still, from the following products : cystine and its derivatives, lysine and its derivatives, oses, and their hydrogenated derivatives, or other polyols (glycerol).

**10.** Composition, characterized in that it is composed of a mixture of at least two of the products according to any one of Claims 1 to 9.

**11.** Composition according to Claim 10, characterized in that it is formed by a mixture of oligomers of formula **(I)** and in that it has an $F_{mean}$ greater than or equal to 2.

**12.** Process for the preparation of a product according to any one of Claims 1 to 7, characterized in that it essentially

comprises carrying out :

**a)** -a polycondensation between :

- a reactant of formula **A** :

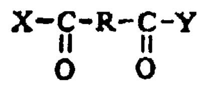

  - with X and Y, which are identical or different, representing a halogen, preferably chlorine, or an -$OR_8$ radical, in which $R_8$ corresponds to hydrogen or to an aliphatic and/or alicyclic radical,
  - and with R in accordance with the definition given in Claim 1,

- and a reactant of formula **B** :

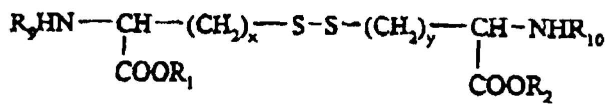

  - with $R_1$ and $R_2$ as defined in Claim 1,
  - with $R_9$ and $R_{10}$, which are identical or different, chosen from the following radicals : H or aliphatic, such as alkyl, hydrogen being particularly preferred,
  - and with x and y = 1 or 2,

**b)** - a reduction of the polymer obtained, which may or may not be subsequently converted.

**13.** Process according to Claim 12, characterized in that the ester functions of the polymer obtained on conclusion of the stage **a)** are hydrolyzed.

**14.** Process according to Claim 12 or 13, characterized in that, prior to the stage **b)**, the at least partially saponified polymer is crosslinked, the crosslinking taking place via at least one bridging agent and, preferably, in the presence of a coupling agent, so as to obtain the network of the type of those according to Claim 9.

**15.** Process according to Claim 14, characterized in that the products according to at least one of Claims 1 to 6 are hydrolyzed so that $R_1$ and $R_2$ = OH.

**16.** Process for the preparation of the polymers according to Claim 8 and/or of the networks according to Claim 9, characterized in that the products obtained from the process according to any one of Claims 12 to 15 are oxidized, preferably using an oxidizing system preferably composed of iodine and/or its derivatives and/or of hydrogen peroxide.

**17.** Biomaterial, characterized in that it is composed of at least one of the products, polymers, networks and compositions according to at least one of Claims 1 to 11.

**18.** Biomaterial, characterized in that it is composed of at least one of the products, polymers, networks and compositions according to at least one of Claims 1 to 11 and of an oxidizing system.

**19.** Biomaterial, characterized in that it is composed of a mixture and/or a formulation of at least one of the products and/or polymers and/or networks and/or compositions according to at least one of Claims 1 to 11 with biological macromolecules or biodegradable synthetic or natural polymers, such as :

- polysaccharides; e.g. starch, cellulose, chitosan, dextran or mucopolysaccharides, such as hyaluronic acid or chondroitin sulfate ;
- proteins; e.g. collagen, gelatin, albumin or globulins ;

- poly(amino acid)s ;
- polyesters (in particular lactic and/or glycolic polyesters), polyorthoesters, polyanhydrides or polyphosphazenes ;
- and lipids and phospholipids.